Europäisches Patentamt

European Patent Office

Office européen des brevets

(11) Publication number: **0 615 972 A1**

# EUROPEAN PATENT APPLICATION

(21) Application number: **93200820.4**

(22) Date of filing: **19.03.93**

(51) Int. Cl.5: **C07H 13/06**

(43) Date of publication of application:
**21.09.94 Bulletin 94/38**

(84) Designated Contracting States:
**AT BE CH DE DK ES FR GB GR IE IT LI LU MC NL PT SE**

(71) Applicant: **UNILEVER N.V.**
**P.O. Box 760**
**NL-3000 DK Rotterdam (NL)**

(72) Inventor: **Van Buuren, Jan**
**Krekweg 8**
**NL-3155 EC Maasland (NL)**
Inventor: **Rosier, Otto Eduard**
**Visserskreek 76**
**NL-3206 GR Spijkenisse (NL)**
Inventor: **Ganguli, Keshab Lal**
**Ribeshof 20**
**NL-2665 GR Bleiswijk (NL)**

(74) Representative: **de Bruijn, Leendert C. et al**
**Nederlandsch Octrooibureau**
**Scheveningseweg 82**
**P.O. Box 29720**
**NL-2502 LS Den Haag (NL)**

(54) Process for refining crude sucrose fatty acid polyester products.

㉗ The invention concerns a process for refining crude sucrose fatty acid polyester reaction product involving a bleaching treatment, which process provides a refined polyester material wherein the level of intermediate molecular weight components is less than 0.2 % calculated by weight of said polyester, said intermediate molecular weight being defined as between 0.4 and 0.65 times the molecular weight of the fully esterified sucrose fatty acid polyester. The bleaching treatment comprises the use of at least one of the following bleaching agents:
(a) mild acid activated bleaching earth, used in the substantial absence of water;
(b) thermally activated bleaching earth, used in the substantial absence of water;
(c) non-activated silica material;
(d) acid activated carbon, used in the substantial absence of water.

The present invention relates to a process for refining crude sucrose fatty acid polyester reaction products, and in particular, although not exclusively, to the bleaching step in such a refining process and to bleached sucrose fatty acid polyester reaction products having a reduced level of minor components.

Sucrose fatty acid polyesters are known as suitable low-calorie fat-replacers in edible products. While they are substantially indigestible for human beings, they have physical and organoleptic properties very similar to those of triglyceride oils and fats conventionally used in edible products. In addition, sucrose fatty acid polyesters are reported to have use as pharmaceutical agents e.g. in view of their ability to take up fat-soluble substances, such as in particular cholesterol, in the gastro-intestinal tract, and subsequently remove these substances from the human body.

The term "sucrose fatty acid polyester" is intended to include any such polyesters or mixtures thereof which consist of 70 % by weight or more of octaesters, i.e. fully esterified sucrose fatty acid esters. In such polyesters, on average about 7.5 or more of the 8 sucrose hydroxyl groups have been esterified with fatty acids, i.e. they have degrees of esterification of about 93.75 % or more.

The term "fatty acid" refers to $C_8$-$C_{24}$ fatty acids which may be saturated or unsaturated, and may have straight or branched alkyl chains. Preferably $C_{14}$-$C_{22}$ fatty acids are present.

In general sucrose fatty acid polyesters are synthesized by a process in which sucrose is reacted with a fatty acid lower alkyl ester, in particular the fatty acid methyl ester, in the presence of a transesterification catalyst, such as an alkali metal hydroxide or carbonate, and an emulsifier, such as an alkali metal soap. In a first stage a sucrose fatty acid mono- or oligoester is formed, which in a second stage is further reacted with the fatty acid lower alkyl ester to form polyesters of the desired degree of esterification. It is also possible to combine the two stages of the reaction into a single step.

Processes of this type have been described in e.g. US patent specifications 3,963,699, 4,517,360, and 4,518,772, and EP patent specifications 256,585, 301,634 and 320,043.

The crude sucrose fatty acid polyester reaction products resulting from conventional syntheses contain, in addition to the desired polyesters, components such as fatty acid soaps, excess fatty acid lower alkyl esters and sucrose fatty acid oligoesters. Also, due to the relatively high temperatures at which conventional processes are carried out, often by-products are formed which may be undesirable in view of their chemical characteristics, such as in particular discolouring properties. In general it is therefore necessary to further purify or refine the crude sucrose fatty acid polyester reaction products resulting from such conventional syntheses.

The term "crude sucrose fatty acid polyester reaction product" is intended to refer to unrefined or partially refined reaction products of processes for the synthesis of sucrose fatty acid polyesters. The two volume-wise major components in such crude compositions in general are the sucrose fatty acid polyester component and the organic solvent.

For the purposes of the present specification the term "organic solvent" is intended to refer to the group of relatively volatile fatty acid sources as may be used in excess amount in the synthesis reaction of the sucrose fatty acid polyester, and any organic solvents used or introduced during synthesis or refining. The major relatively volatile fatty acid source is fatty acid lower alkyl ester used in the transesterification reaction described above.

In the crude reaction product the sucrose fatty acid polyester component is generally present in an amount of 10 to 95 % by weight of the crude product, and the organic solvent is generally present in an amount of 5 to 90 % by weight.

Conventional refining methods comprise a variety of different treatments including washings with water, organic solvents, acid or alkaline solutions, salting-out treatments, bleaching steps, distillation, stripping and deodorization treatments.

In general the washing treatments aim at a substantial reduction of the soap component and colour which can be present in the crude sucrose fatty acid polyester reaction product as a result of its use as the emulsifier system in the preceding synthesis reaction of the polyester or, to a limited extent, due to partial conversion to soap of the fatty acid source, such as the fatty acid lower alkyl ester.

These washing treatments are often followed by a bleaching treatment with adsorbents such as bleaching earth, activated carbon and silicas, primarily aimed at removing coloured matter, discolouring materials, residual soap and metal ions. Descriptions thereof can be found in US-A-4,334,061, EP-A-319,091, EP-A-319,092, EP-A-434,117, EP-A-434,119 and EP-A-435,364.

It has been found that one group of components in the refined and bleached sucrose fatty acid ester mixture consists of organic materials having a molecular weight which is between the average molecular weight of the sucrose fatty acid ester and the molecular weight of triglyceride materials often used for the production of the sucrose fatty acid esters. It is believed that the level of these components increases during the bleaching and/or distillation, stripping and deodorization. The extent of increase is found to depend mainly on the type of absorbents/bleaching materials. Since the sucrose fatty acid esters may be intended for use in foods and pharmaceutical products, it is desirable to minimize the level of minor components in the composition.

It is therefore a first object of the present invention to provide a treatment suitable for the refining of crude sucrose fatty acid polyester reaction products which result in a reduced level of these intermediate molecular weight components.

Accordingly, the present invention in its broadest aspect provides a process for refining crude sucrose fatty acid polyester reaction product, comprising the step of subjecting the reaction product to a bleaching treatment, characterized in that it comprises the step of providing a bleached polyester material wherein the level of intermediate molecular weight components is less than 0.2 % calculated by weight of said polyester.

In this specification the term intermediate molecular weight (IMW) components includes any organic compound having a chromatography peak or shoulder which is situated between the chromatography peak(s) or shoulder(s) corresponding to the sucrose fatty acid polyester(s) and the chromatography peak(s) or shoulder(s) corresponding to the triglyceride starting material(s). Such chromatography peaks may be obtained by any suitable method of chromatography, for example capillary supercritical fluid (SFC) or gel permeation chromatograms (GPC). For the purpose of the invention SFC is the preferred technique.

The content of IMW components in the sucrose polyester appears also to be related to the para-anisidine value (PAV). Thus, the bleached polyester obtained by the process of the invention preferably has a PAV of 5 or lower.

Alternatively, the intermediate molecular weight is defined with reference to the molecular weight of the fully esterified sucrose, i.e. the sucrose fatty acid octaester: the IMW components are defined herein as having a molecular weight between 0.4 and 0.65 times the molecular weight of sucrose octaester. Table I shows the molecular weight ranges of the IMW components in relation to the chain length of the saturated fatty acid residues in the corresponding sucrose polyesters.

Table I

| Average number of C-atoms in fatty acid residues | MW sucrose octaester | MW range IMW components |
|---|---|---|
| 8 | 1351.9 | 541 - 879 |
| 10 | 1576.3 | 631 - 1025 |
| 12 | 1800.8 | 720 - 1171 |
| 14 | 2025.2 | 810 - 1316 |
| 16 | 2249.6 | 900 - 1462 |
| 18 | 2474.1 | 990 - 1608 |
| 20 | 2698.5 | 1079 - 1754 |
| 22 | 2922.9 | 1169 - 1900 |
| 24 | 3147.4 | 1259 - 2045 |

Preferably the level of IMW materials is less than 0.2 % by weight, more preferably less than 0.1 % by weight, most preferably less than 0.05 % by weight.

Another aspect of the present invention is that the above mentioned process for the first time allows the preparation of bleached sucrose fatty acid esters having a low level of IMW materials. Therefore the invention also relates to compositions comprising a sucrose fatty acid ester material and less than 0.2 % based on the weight of the sucrose fatty acid ester material of IMW materials. Generally the level of sucrose fatty acid ester materials in these compositions is at least 10% by weight, preferably 20-100%, more preferredly 50-100%, most preferredly 80-100%.

Preferably, the refining of the crude sucrose fatty acid polyester product comprises, in addition to the above-mentioned bleaching step, a distilling step by which the organic solvent and optionally further volatile components such as e.g. free fatty acids present in the crude reaction product are removed to a substantial degree. This distilling step may take place before or after the bleaching step, although it is preferred that the distilling takes place before the bleaching treatment. Best results are achieved when the organic solvent is substantially removed from the crude reaction product prior to a subsequent bleaching treatment, but worthwhile advantages are already obtained at removal levels of the organic solvent of 40 % by weight or more, removal levels of 70 % or more being preferred, and levels of 95 % or more being preferred most.

3

The optional distilling step is preferably carried out after one or more preliminary refining treatments of the crude reaction mixture as set out below.

The optional distillation of the crude reaction product may be carried out in a conventional manner using temperatures in the range of from 60 to 300°C. In general temperatures of above 150°C will be used since these allow reduced pressure regimes which are economically feasible on a technical scale. Preferred distilling temperatures lie within the range of 160 to 240°C, temperatures of 200 to 240°C being preferred most.

As already indicated, such a distilling step is preferably carried out under reduced pressure, in particular pressures of below 100 mbar or rather below 70 mbar. Pressures of between 1 and 10 mbar and in particular pressures between 1 and 5 mbar are preferred.

Although appropriate distilling times will strongly depend upon the temperature and pressure conditions applied, batch-wise distilling times of between 30 and 100 minutes have been found to be suitable, whereas continuous distilling times can be much shorter and are determined by the equipment used.

In exceptional circumstances where the crude reaction product at the distilling stage still comprises significant levels of relatively temperature-sensitive components, it may be necessary to use relatively low distilling temperatures within the range of 60 to 150°C, in particular 80 to 120°C. Removal from or reduction in the crude reaction product of the relevant organic components by distilling at such low temperatures will require further reduced pressures, in particular of below 1 mbar, or even below 0.1 mbar.

The bleaching treatment and the bleaching agent selected are particularly triggered towards effective minimization of the level of intermediate molecular weight materials. It has been found that several conventional bleaching agents and/or bleaching conditions are not capable of a suitable minimization of the level of intermediate molecular weight materials. For the purpose of the present invention it is therefore preferred to use a combination of bleaching agent and bleaching conditions which advantageously results in low levels of intermediate molecular weight materials.

It has been found that advantageously, though not exclusively, the following bleaching treatments may be used:

(a) mildly acid activated bleaching earth, used in the substantial absence of water;

(b) thermally activated bleaching earth, used in the substantial absence of water;

(c) non-activated silica materials;

(d) acid activated carbon, used in the substantial absence of water.

As indicated above, the bleaching with mildly acid activated or thermally activated bleaching earth or with acid activated carbon should be carried out in the substantial absence of water. For the purpose of the invention the term substantial absence of water means a level of water during bleaching of less than 2% by weight of the sucrose fatty acid ester material, more preferably less than 0 to 1%, most preferably from 0 to 0.1%. A bleaching treatment with non-activated silica materials may be carried out either in the presence or in the absence of water, preferably however bleaching with these materials also takes place in the substantial absence of water.

For the purpose of the invention, mildly acid activated bleaching earth is a bleaching earth which has undergone a treatment with acids such that it is only moderately acidic. Moderately acidic means here that a 10 % aqueous slurry of the bleaching earth at 25°C has a pH of at least 3.75, in particular of at least 4.0. Alternatively, a mildly acid activated bleaching earth can be defined as being milder than the commercially available mild activated bleaching earth known as Standard FF ex Tonsil, i.e. the pH of an aqueous slurry having a value higher than that of Standard FF, or the removal capacity for chlorophyll or phosphorus from rapeseed oil under standard conditions not exceeding than that of Standard FF.

Thermally activated bleaching earth is any bleaching earth which has undergone a thermal treatment e.g. at 700 to 800°C. An example of suitable material is a calcinated metal oxide-silica absorbent. This absorbent can be regenerated by calcination without substantial loss of activity. A suitable thermally activated bleaching earth is a synthetic bleaching earth as described in EP-A-269,173, US-A-4,812,436 and US-A-4,956,126. Preferably, a 10 % aqueous slurry of the thermally activated bleaching earth at 25°C also has a pH of at least 3.75, in particular of at least 4.0.

Non-activated silica or neutral silica is a silica material which has not been acidified and/or chemically treated. Examples of suitable materials are amorphous precipitated silica, e.g. Trisyl and Trisyl 300 having water contents of more than 30%, preferably 60-70 wt% (hydrogels), ex Davison Chemical Division of W.R. Grace & Co as described in EP-A-389,057. Another example is a precipitated amorphous silica having a surface area of at least 400 $m^2/g$ and a pore size of at least 2 nm and containing virtually no water; an example of such a material is macroporous silica (Masil) ex Joseph Crosfield Ltd, as decribed in EP-A-361,622. The latter material has been found to be especially suitable for carrying out the process of the invention. In general, a 10 % aqueous slurry of the non-activated or neutral silica at 25°C will have a pH of at least 3.75, or even 4.0 or higher.

For the purpose of the invention acid activated carbon materials are granular or powdered carbon materials which have been activated by means of an acid treatment; suitable materials are for example CA1, CA2, CA3, CA4, CA5 and CN1 ex Norit.

Suitably, the bleaching agents are added to the reaction product in amounts of 0.2 to 5 % by weight. Higher amounts can also be used, but in general no further improvements are obtained beyond 5 %. Preferred amounts are of 0.5 to 3 % by weight. Often very good results are obtained with amounts of 1 to 2 % by weight.

In general the bleaching treatment is preferably carried out at an elevated temperature. Preferred bleaching temperatures are within the range of 70 to 140°C, temperatures of 80 to 120°C being particularly preferred.

Where bleaching is best carried out under dry conditions, suitably conditions of reduced pressure are applied, pressures of below 100 mbar and in particular between 30 and 70 mbar being preferred.

Normal bleaching times vary between 30 and 120 minutes, times of between 30 and 60 minutes in many instances being sufficient.

After the bleaching treatment the bleaching agent together with the absorbed coloured materials are in general removed by filtration, which may be assisted by introducing a filter aid such as cellulose.

The process of the invention may advantageously include two or more separate bleaching treatments using the same or different bleaching agents. If two bleaching steps are performed, at least one of these is preferably carried out using the bleaching agents mentioned under (a) to (d) above; for the other bleaching step conventional adsorbents may also be used. Also, if two bleaching steps are performed, at least one of these is preferably carried out after the distillation treatment. Carrying out at least one bleaching step after distillation allows a more efficient use of bleaching materials. Also a more effective removal of colour is achieved when at least one bleaching step is performed after distillation and even more so if two bleaching steps are used.

In addition to the distilling and bleaching treatment mentioned above, the refining process in accordance with the present invention preferably comprises refining treatments preceding the distilling step to substantially remove the soap and metal ion components in the crude reaction product. Removal of these components will avoid problems of discolouring during subsequent high temperature refining treatments, such as the distilling step. At the stage before the distilling step it is not so much an object to remove the coloured matter and this may suitably be primarily effected during the bleaching step. Substantial removal of soap and metal ions is to be understood as a removal to a residual level of no more than 100 ppm.

These pre-distilling treatments may comprise any suitable method to substantially remove in particular the soap and alkali metal ions which may be present in the crude reaction product. Suitably, such refining treatments include conventional washing treatments such as water washings with or without added electrolytes, and alkaline or acid washings as described in EP-A-319,092 herein incorporated by reference. Advantageously the pretreatment comprises an alkaline washing, e.g. with aqueous soda, and one or more water washings.

Alternatively, it may be useful to contact the crude polyester reaction product with an acid to substantially convert the soap component to its corresponding free fatty acids. The acid is used to convert the soap component to its free fatty acids and the amount thereof in principle must be sufficient to substantially convert all of the soap present in the crude polyester product. The amount of acid will depend upon the level of the soap emulsifier system used in the synthesis reaction as well as upon the amounts of soap formed or introduced during the synthesis reaction. For reasons of cost and in order to avoid acid hydrolysis of the polyester product, it is preferred that the amount of acid is as close to the precise amount needed as technically feasible to ensure full neutralising of all the soap present in the crude reaction product.

To ensure substantially full conversion of the soap to free fatty acids the strength of the acid must be such that the equilibrium of the conversion reaction lies substantially fully at the side of the free fatty acids, the pH values resulting from the addition of the acid to the crude reaction product, in general in the form of an aqueous solution thereof, preferably being below 6, the range of pH 3 to 5 being preferred most; the latter corresponds to a neutralization factor (ratio of moles of acid to moles of soap plus moles of lye) of between about 0.8 and 1.

Suitably, both inorganic and organic acids can be used which in view of the important application of the sucrose fatty acid polyesters in food products preferably are food grade. Suitable inorganic acids are phosphoric acid and alkali metal dihydrogen phosphates. Suitable organic acids include acetic, lactic, succinic and citric acid, the latter acid being preferred. Preferably, a relatively concentrated aqueous acid solution is used. Suitable concentrations lie within the range of 25 % to 85 % by weight, 40 to 60 % being preferred most.

The acid step is followed by substantial removal from the crude polyester reaction product of any salts present therein, in general alkali metal salts which together with the free fatty acids result from the soap conversion by the acid. The substantial removal of the salt may be effected by using conventional separation techniques, such as centrifuge or filtration techniques. Suitable filtration techniques may involve the use of filter aids, such as e.g. cellulose.

In particular, when the salt is removed by way of filtration, it has been found that the removal of this salt, generally being the alkali metal salt of the acid used in the soap conversion step, is improved if the water level in the acidulated reaction product resulting e.g. from the aqueous acid solution, is reduced to very low levels which preferably correspond to a system substantially without free water being present, i.e. all water being either dissolved in the polyester phase or being present as crystal or bound water of further components in the reaction product. Suitably, such water levels are below 0.3 % by weight, and preferably below 0.1 or even 0.05 % by weight. This can be conveniently effected by subjecting the reaction product to drying at elevated temperature and/or reduced pressure. This drying step may be carried out subsequent to or during the contact times discussed above.

A preferred method of reducing the water to very low levels is flash-drying by which the reaction product is passed into a low pressure chamber and any water present is vaporized adiabatically. The heat needed for such evaporation is drawn from the reaction mixture and accordingly this method can be used for the simultaneous drying and cooling of the reaction mixture from the temperature of the acid step to below 100°C, in particular 70 to 90°C. If flash-drying is used for simultaneous drying and cooling, the water level in the reaction mixture after the addition of the acid solution may be higher than described above, in order to ensure sufficient cooling during the flash-drying process. Water levels of 2 to 5 % by weight will ensure a cooling of about 20 to 50°C which in general will avoid any further separate cooling step.

It has further been found that the removal of the salt and also the colour properties of the final refined polyester product are advantageously affected, if prior to the introduction of the acid in the pre-distilling treatment a relatively small amount of an aqueous alkaline solution is added to the crude polyester product which is subsequently neutralized by the acid simultaneous to the conversion of the soap component to its free fatty acid.

Within the constraint of minimizing the risk of the formation of undesirable components, the particular combination of source, volume and level of alkalinity is not very critical and can be any of the readily available alkaline materials, such as the alkali metal hydroxides, carbonates or silicates, generally at a level within the range of 0.1 to 6 N, in particular 0.2 to 2.5 N. The aqueous alkaline solution is suitably added to the crude polyester product in an amount of 0.5 to 5 % by weight of the product. Preferably, some agitation is applied to improve the contact between the crude polyester product and the aqueous alkaline solution. In a batch-wise operation contact times in the acid step and optional prior alkaline step of between 1 and 10 minutes between the introduction of the aqueous alkaline solution and the introduction of the acid have been found sufficient. In a continuous operation contact times are generally shorter than 3 minutes, such as less than about 1 minute, and can be as short as 5 to 30 seconds.

In the predistilling refining treatment the acid step and optional prior alkaline step can be combined with further conventional washing steps as described hereinbefore.

The invention is directed at refining crude sucrose fatty acid polyester reaction products of transesterification reactions involving excess amounts of fatty acid lower alkyl esters. This type of transesterification is particularly suitable for the synthesis of sucrose fatty acid polyesters consisting for 70 % or more of sucrose octaesters. Accordingly, the present process is particularly applicable to the refining of crude sucrose fatty acid polyester having high degrees of esterification, in particular crude products comprising sucrose fatty acid polyesters having degrees of esterification of 93.75 % or more, or even 95 % or more.

Having a reduced risk of discolouring the sucrose fatty acid polyesters refined in accordance with the process of the present invention are particularly suitable to replace fully or partially conventional triglyceride fats in food compositions intended for high temperature purposes, such as baking and frying oils. Generally, in such food compositions at least 10 % by weight of the conventional triglyceride fat is replaced by the sucrose fatty acid polyesters produced in accordance with the present invention. Preferably, at least 50 % of the conventional fat is replaced by the polyesters.

The invention is now further illustrated with reference to the following examples, percentages being by weight unless indicated otherwise.

EXAMPLE 1

A batch of crude sucrose fatty acid polyester reaction product, synthesized in a solvent-free transesterification reaction between sucrose and 53% fully hardened palm oil (PO) and 47% fully hardened palm kernel oil (PKO) derived, distilled fatty acid methyl ester to a degree of esterification of over 95%, consisted of the following components:

| | | |
|---|---|---|
| sucrose fatty acid polyester | | 52.3 % |
| fatty acid methyl ester | | 39.7 % |
| soap (mainly coconut-derived potassium soap) | | 5.2 % |
| minor components | | 2.8 % |
| Level of IMW components | | |
| GPC (including triglycerides) | 1.4 % | |
| SFC (not including triglycerides) | | <0.02 % |

This batch was dried under 50 mbar at 90°C while agitating and then 4% of 0.8N NaOH was added and agitated for 5 minutes at 90°C under atmospheric pressure. To this mixture sufficient $H_3PO_4$ (50% solution) was dosed to neutralize all soap present in the oil phase and NaOH in the water phase. This mixture was centrifuged at 3000 rpm for 10 minutes at 80°C to separate aqueous phase from the oil. The oil phase was washed with 5% water and the mixture was centrifuged at 3000 rpm for 10 minutes at 80°C.

| Level of IMW Components (%) | GPC | SFC |
|---|---|---|
| After 5% water wash | 1.4 | <0.02 |

The water washed sucrose polyester was further refined using five different procedures. Each batch was dried under 50 mbar at 90°C while agitating. Fifteen grams of a bleaching agent as specified below were then added per kg of sucrose polyester and the resulting mixture was stirred at 90°C. The mixture was filtered through a 2 microns filter and distilled for 1 hour at 200°C under 1 mbar and then deodorized for 3 hours at 215°C under 4 mbar.

procedure 1 : 15 grams of Standard FF. ex Tonsil[R] were added and the mixture was stirred for 30 minutes under 50 mbar.

| | | |
|---|---|---|
| After bleaching/filtration | 1.3 | 0.03 |
| After distillation/deodorization | 2.1 | <0.02 |

procedure 2 : 15 grams of synthetic bleaching earth ex Joseph Crosfield Ltd. were added and the mixture was stirred for 30 minutes under atmospheric pressure and another 30 minutes under 50 mbar.

| | | |
|---|---|---|
| After bleaching/filtration | 1.4 | <0.02 |
| After distillation/deodorization | 2.0 | <0.02 |

procedure 3 : 15 grams of macroporous silica ex Joseph Crosfield Ltd. were added and the mixture was stirred for 30 minutes under atmospheric pressure.

| | | |
|---|---|---|
| After bleaching/filtration | 1.3 | <0.02 |
| After distillation/deodorization | 2.1 | <0.02 |

procedure 4 : 15 grams acidic carbon (CA1 ex Norit[R]) was added and the resulting mixture was stirred for 30 minutes under 50 mbar.

| | | |
|---|---|---|
| After bleaching/filtration | 1.3 | <0.02 |
| After Distillation/Deodorization | 2.1 | <0.02 |

procedure 5: (comparison) 15 grams of bleaching earth (Supreme FF. ex Tonsil[R]) was added and the resulting mixture was stirred for 30 minutes under 50 mbar.

| | | |
|---|---|---|
| After bleaching/filtration | - | 0.1 |
| After distillation/deodorization | - | 0.1 |

EXAMPLE 2

A batch of crude sucrose fatty acid polyester reaction product, synthesized in a solvent-free transesterification reaction between sucrose and 53% PO and 47% PKO (cf. Example 1) derived, distilled fatty acid methyl ester to a degree of esterification of over 95%, consisted of the following components:

| | | |
|---|---|---|
| sucrose fatty acid polyester | | 54.0 % |
| fatty acid methyl ester | | 40.2 % |
| soap (mainly coconut-derived potassium soap) | | 4.4 % |
| minor components | | 1.3 % |
| Level of IMW components (GPC) | 0.67 % | |

7.5% of aqueous sodium carbonate solution was dosed into this batch of crude sucrose polyester and stirred for 10 minutes at 85°C and 200 rpm under atmospheric pressure. The batch was then continuously centrifuged at 9600 rpm at 85°C.

| Level of IMW Components (%) | GPC |
|---|---|
| After washing with sodium carbonate : | 0.9 |

The sucrose polyester phase was then washed with 10% water at 85°C while stirring at 200 rpm for 10 minutes. This mixture was then continuously centrifuged 9600 rpm and at 85°C.

| After water washing / centrifuging | 0.9 |
|---|---|

The sucrose polyester phase was then further refined according to the following procedure. The oil phase was dried under 10 mbar at 85°C for 20 minutes. 2 grams of water per kg of this dried mixture were added and stirred for 10 minutes at 85°C under atmospheric pressure. 10 grams of adsorbents (Trisyl having water content not less than 30% and preferably 60-70 % ex Davison Chemical Division of W.R. Grace & Co[R]) per kg of this mixture were dosed and agitated for 30 minutes at 85°C under atmospheric pressure, the mixture was then filtered over a plate and frame filter.

| After Trisyl treatment/filtration | 0.7 |
|---|---|

2 grams of water per kg of this mixture were added and stirred at 200 rpm for 10 minutes at 85°C under atmospheric pressure. 15 grams of bleaching earth (Supreme FF. ex Tonsil[R]) per kg of this mixture were added to this mixture and agitated at 200 rpm at 85°C for 30 minutes under atmospheric pressure and then another 30 minutes under 10 mbar. The mixture was then filtered over plate and frame filter.

| After Supreme FF treatment/filtration | 3.1 |
|---|---|

The mixture was then distilled for 1 hour under 1 mbar and deodorized at 200°C for 3 hours.

| After distillation/deodorization | 5.6 |
|---|---|

EXAMPLE 3

A batch of crude sucrose fatty acid polyester reaction product, synthesized in a solvent-free transesterification reaction between sucrose and 53% PO and 47% PKO (cf. Example 1) derived, distilled fatty acid methyl ester to a degree of esterification of over 95%, consisted of the following components:

| | | |
|---|---|---|
| sucrose fatty acid polyester | | 55 % |
| fatty acid methyl ester | | 38.8 % |
| soap (mainly coconut-derived potassium soap) | | 4.2 % |
| minor components | | 2.0 % |
| Level of IMW components (GPC) | 0.95 % | |

7.5% aqueous sodium carbonate solution was dosed into this batch and stirred for 10 minutes at 85°C and 200 rpm under atmospheric pressure. The batch was then continuously centrifuged at 9600 rpm and at 85°C.

The sucrose polyester phase was further refined according to the following procedure. 2 grams of water per kg were dosed and stirred for 10 minutes at 200 rpm under atmospheric pressure at 85°C. Into this mixture, 15 grams of macroporous silica ex Joseph Crosfield Ltd. per kg of the mixture were dosed and agitated for 30 minutes under atmospheric pressure at 200 rpm. This mixture was filtered over plate and frame filter. This mixture was distilled at 200°C under 4 mbar for 1.5 hours and then the mixture was deodorized for 4 hours at 200°C under 4 mbar.

| Level of IMW Components | GPC % | SFC % |
|---|---|---|
| After distillation/deodorization | <0.02 | <0.02 |

EXAMPLE 4

A batch of crude reaction product, synthesized in a solvent-free transesterification between sucrose and touch-hardened soybean oil derived, distilled methyl ester to a degree of esterification of over 95%, consisted of the following components :

| | | |
|---|---|---|
| sucrose fatty acid polyester | | 51.3% |
| fatty acid methyl ester | | 44.1% |
| soap (mainly coconut-derived potassium soap) | | 3.1% |
| minor components | | 1.5% |
| Level of IMW components | GPC | SFC |
| | <0.02% | <0.02% |

This batch was first dried under 50 mbar at 90°C for 10 minutes. Per kg of crude sucrose polyester 40 grams of 0.8 N aqueous NaOH were added and the mixture was stirred at 600 rpm at 90°C for 5 minutes under atmospheric pressure. Sufficient 50% aqueous $H_3PO_4$ was added and agitated for 1 minute at 1500 rpm at 90°C to neutralize all soap present in the polyester phase and all NaOH present in the water phase. This mixture was centrifuged batchwise for 15 minutes at 3500 rpm and 80°C. This partially refined sucrose polyester after separating from the water phase by centrifuge was dried under 50 mbar pressure at 90°C for 10 minutes. The sucrose polyester was further refined by two different procedures.

Procedure 1 : wet adsorbent treatment and dry bleach

2 grams of water per kg of this partially refined sucrose polyester were added and stirred for 5 minutes at 600 rpm at 90°C under atmospheric pressure. Then 5 grams of adsorbents (Trisyl having water content not less than 30% and preferably 60-70% ex Davison Chemical Division of W.R. Grace & Co[R]) per kg of this mixture were dosed and stirred for 30 minutes at 600 rpm, 90°C under atmospheric pressure. The mixture was then filtered over a 2 micrometer filter.

| Level of IMW Components | GPC % | SFC % |
|---|---|---|
| After Trisyl treatment/filtration | <0.02 | <0.02 |

After filtration, the partially refined sucrose polyester was batch-distilled for 1 hour at 215°C under 1 mbar pressure.

| After distillation | 0.4 | 0.04 |
|---|---|---|

This batch of distilled sucrose polyester was dried under 50 mbar at 90°C for 10 minutes. 15 grams of bleaching earth (Standard FF. ex Tonsil[R]) per kg of distilled sucrose polyester were dosed and stirred for 30 minutes at 600 rpm, 90°C under atmospheric pressure. The mixture was filtered over 2 micrometer filter. This batch of filtered sucrose polyester was then batch deodorized for 2 hours at 215°C under 4 mbar pressure.

| After deodorization | 0.6 | 0.12 |
|---|---|---|

Procedure 2 : Dry prebleach and dry bleach
10 grams of Supreme FF. ex Tonsil[R] per kg of the refined sucrose polyester were dosed to this mixture and stirred for 30 minutes at 600 rpm, 90°C under atmospheric pressure. The mixture was then filtered over a 2 micrometer filter.

| After Supreme FF treatment/filtration | 0.2 | 0.08 |
|---|---|---|

This filtered batch of partially refined sucrose polyester was batch-distilled for 1 hour at 200°C under 1 mbar pressure.

| After distillation | 0.5 | 0.06 |
|---|---|---|

This batch of distilled sucrose polyester was dried under 50 mbar at 90°C for 10 minutes. 10 grams of Standard FF. ex Tonsil[R] per kg of distilled sucrose polyester were dosed and stirred for 30 minutes at 600 rpm, 90°C under atmospheric pressure. The mixture was filtered over 2 micrometer filter. This batch of filtered sucrose polyester was then batch-deodorized for 2 hours at 200°C under 4 mbar pressure.

| Level of IMW components | GPC % | SFC % |
|---|---|---|
| After deodorization | 0.7 | 0.14 |

EXAMPLE 5

A batch of crude sucrose fatty ester polyester synthesized and having the composition according to Example 4 was partially refined according to the process of Example 4, with the exception that after acid treatment and centrifugation, the mixture was washed with 10% water for 10 minutes and centrifuged again for 15 minutes at 3500 rpm at 80°C in order to separate the water phase.
This partially refined sucrose polyester was further refined by two different procedures.
Procedure 1 : wet adsorbent treatment and dry bleach (Split Bleach)
The mixture was dried at 90°C under 50 mbar. 2 grams of water per kg of partially refined sucrose polyester was added and stirred for 5 minutes at 600 rpm, 90°C under atmospheric pressure. Then 5 grams of macroporous silica ex Joseph Crosfield Ltd. per kg of partially refined sucrose polyester were dosed to this mixture and stirred for 30 minutes at 600 rpm, 90°C under atmospheric pressure. The mixture was then filtered over a 2 micrometer filter.

| Level of IMW Components | GPC % | SFC % |
|---|---|---|
| After silica treatment/filtration | <0.02 | <0.02 |

After filtration, the partially refined sucrose polyester was batch-distilled for 2 hours at 190°C under 1 mbar pressure.

| After distillation | 0.4 | 0.04 |
|---|---|---|

This batch of distilled sucrose polyester was dried under 50 mbar at 90°C for 10 minutes. 15 grams of acidic carbon (CA1 ex Norit[R]) per kg of distilled sucrose polyester were dosed and stirred for 30 minutes at 600 rpm, 90°C under 50 mbar pressure. The mixture was filtered over a 2 micrometer filter. This batch of filtered sucrose polyester was then batch-deodorized for 4 hours at 190°C under 4 mbar pressure.

| After deodorization | 0.6 | 0.12 |
|---|---|---|

Procedure 2 : Post Bleach

The partially refined sucrose polyester was batch-distilled for 2 hours at 190°C under 1 mbar pressure. The mixture was dried at 90°C under 50 mbar. 2 grams of water per kg of this partially refined sucrose polyester were added and stirred for 5 minutes at 600 rpm, 90°C under atmospheric pressure. Then 5 grams of macroporous silica ex Joseph Crosfield Ltd. per kg of partially refined sucrose polyester was dosed to this mixture and stirred for 30 minutes at 600 rpm, 90°C under atmospheric pressure. The mixture was then filtered over a 2 micrometer filter.

| After silica treatment/filtration | <0.02 | <0.02 |
|---|---|---|

This batch of sucrose polyester was dried under 50 mbar at 90°C for 10 minutes. 15 grams of acidic carbon (CA1 ex Norit[R]) per kg of distilled sucrose polyester were dosed and stirred for 30 minutes at 600 rpm, 90°C under 50 mbar. The mixture was filtered over a 2 micrometer filter. The filtered sucrose polyester was then batch-deodorized for 4 hours at 190°C under 4 mbar pressure.

| After deodorisation | <0.02 | <0.02 |
|---|---|---|

EXAMPLE 6

A batch of crude reaction product, synthesized in a solvent-free transesterification between sucrose and touch-hardened soybean oil derived, distilled methyl ester to a degree of esterification of over 95%, consisted of the following components :

| | | |
|---|---|---|
| sucrose fatty acid polyester | | 49.0% |
| fatty acid methyl ester | | 45.2% |
| soap (mainly coconut-derived potassium soap) | | 4.7% |
| minor components | | 1.1% |
| Level of IMW components (GPC) | <0.02% | |

This batch of crude sucrose polyester was first dried under 50 mbar at 90°C for 10 minutes. Per kg of crude sucrose polyester 40 grams of 0.8N aqueous NaOH were added and stirred at 600 rpm at 90°C for 5 minutes under atmospheric pressure. Sufficient aqueous $H_3PO_4$ (50%) was added and agitated for 1 minute at 1500 rpm at 90°C to neutralize all soap present in the SPE phase and all NaOH present in the water phase. The mixture was centrifuged batchwise for 15 minutes at 3500 rpm and 80°C. The partially refined sucrose polyester after separating from the water phase by centrifuge was dried under 50 mbar pressure at 90°C for 10 minutes. This partially refined sucrose polyester was further refined by six different procedures.

Procedure 1 : Wet bleach
2 grams water per kg of partially refined sucrose polyester was added and stirred for 5 minutes at 1790 rpm, 90°C under 1 bar. Then 15 grams of macroporous silica ex Joseph Crosfield Ltd. per kg of partially refined sucrose polyester were added and stirred for 30 minutes at 1790 rpm, 90°C under atmospheric pressure. The mixture was then filtered over a 2 micrometer filter.

| | | |
|---|---|---|
| After silica treatment/filtration | <0.02 | <0.05 |

This filtered batch of sucrose polyester was distilled for 1 hour at 215°C under 1 mbar pressure and then deodorized for 3 hours at 215°C under 4 mbar pressure.

| | | |
|---|---|---|
| After distillation/deodorization | 0.8 | 0.1 |

Procedure 2 : Dry bleach
15 grams of synthetic bleaching earth ex Joseph Crosfield Ltd. per kg of partially refined sucrose polyester were dosed and stirred for 30 minutes at 1790 rpm, 90°C under atmospheric pressure and 30 minutes under 50 mbar. The mixture was then filtered over a 2 $\mu$m filter.

| Level of IMW components | GPC% | SFC% |
|---|---|---|
| After bleaching/filtration | <0.02 | - |

This filtered batch of sucrose polyester was distilled for 1 hour at 215°C/1 mbar and then deodorized for 3 hours at 215°C/4 mbar.

| | | |
|---|---|---|
| After distillation/deodorization | 0.6 | 0.1 |

Procedure 3 : Wet bleach
1 gram of water per kg of partially refined sucrose polyester was added and stirred for 5 minutes at 1790 rpm/90°C/1 bar. 15 grams of adsorbent (Trisyl having water content not less than 30% and preferably 60-70% ex Davison Chemical Division of W.R. Grace & Co[R]) per kg of this mixture were dosed and stirred for 30 minutes at 1790 rpm/90°C/ 1 bar. The mixture was then filtered over a 2 $\mu$m filter.

| | | |
|---|---|---|
| After Trisyl treatment/filtration | <0.02 | <0.02 |

This filtered batch of sucrose polyester was distilled for 1 hour at 215°C/1 mbar pressure and then deodorized for 3 hours at 215°C/4 mbar.

| | | |
|---|---|---|
| After distillation/deodorization | 0.5 | 0.1 |

Procedure 4 : Dry bleach
15 grams of acidic carbon (CA1 ex Norit[R]) per kg of partially refined sucrose polyester were dosed and stirred for 30 minutes at 1790 rpm/90°C/1 bar. The mixture was then filtered over a 2 $\mu$m filter.

| Level of IMW components | GPC % | SFC % |
|---|---|---|
| After CA1 treatment/filtration | <0.02 | <0.05 |

This filtered batch of sucrose polyester was distilled for 1 hour at 215°C/1 mbar and then deodorized for 3 hours at 215°C/4 mbar.

| After distillation/deodorization | 0.7 | 0.1 |
|---|---|---|

Procedure 5 : Dry bleach

15 grams of bleaching earth (Standard FF. ex Tonsil$^R$) per kg of partially refined sucrose polyester were dosed and stirred for 30 minutes at 1790 rpm/90°C/50 mbar. The mixture was then filtered over a 2 $\mu$m filter.

| After bleaching treatment/filtration | 0.2 | <0.05 |
|---|---|---|

This filtered batch of sucrose polyester was distilled for 1 hour at 215°C/1 mbar and then deodorized for 3 hours at 215°C/4 mbar.

| After distillation/deodorization | 0.7 | 0.1 |
|---|---|---|

Procedure 6 : Dry bleach (comparison)

15 grams of bleaching earth (Supreme FF. ex Tonsil$^R$) per kg of partially refined (lye/phosphoric acid treated) sucrose polyester were dosed and stirred for 30 minutes at 1790 rpm/90°C/50 mbar. The mixture was then filtered over 2 $\mu$m filter.

| After Supreme FF treatment/filtration | 0.5 | 0.4 |
|---|---|---|

This filtered batch of sucrose polyester was distilled for 1 hour at 215°C/1 mbar and then deodorized for 3 hours at 215°C/4 mbar.

| After distillation/deodorization | 1.1 | 0.4 |
|---|---|---|

EXAMPLE 7

A batch of crude reaction product, synthesized in a solvent-free transesterification between sucrose and touch-hardened soybean oil derived, distilled methyl ester to a degree of esterification of over 95%, consisted of the following components :

| | | |
|---|---|---|
| sucrose fatty acid polyester | | 48.6% |
| Fatty acid methyl ester | | 45.7% |
| Soap(mainly coconut-derived potassium soap) | | 4.2% |
| minor components | | 1.5% |
| Level of IMW components | GPC | SFC |
| | <0.02% | <0.02% |

7.5% aqueous sodium carbonate was dosed into this batch of crude sucrose polyester and stirred for 10 minutes at 85°C/200 rpm/1 bar. The batch was then continuously centrifuged at 9600 rpm at 85°C.

2 grams of water per kg of partially refined refined sucrose polyester were dosed and stirred for 10 minutes at 200 rpm/85°C/1 bar. In three different procedures 15 grams of adsorbent (1: macroporous silica ex Joseph Crosfield Ltd.; 2: L80ff ex Tonsil[R]; 3: Supreme FF. ex Tonsil[R]) per kg of the mixture were dosed and agitated for 30 minutes at 200 rpm/1 bar. The mixture was filtered over a plate and frame filter. This mixture was distilled at 200°C under 1 mbar for 1.5 hours and the mixture was deodorized for 4 hours at 200°C/4 mbar.

| Level of IMW components | GPC % | SFC % |
|---|---|---|
| After distillation/deodorization | | |
| 1: Macroporous silica | 0.5 | 0.1 |
| 2: L80ff (comparison) | 0.4 | 0.8 |
| 3: Supreme FF (comparison) | 1.5 | 1.1 |

EXAMPLE 8

A batch of crude reaction product, synthesized in a solvent-free transesterification between sucrose and touch-hardened soybean oil derived, distilled methyl ester to a degree of esterification of over 95%, consisted of the following components :

| | |
|---|---|
| sucrose fatty acid polyester | 50.6% |
| Fatty acid methyl ester | 42.3% |
| Soap (mainly coconut-derived potassium soap) | 4.5% |
| minor components | 2.6% |
| Level of IMW components (SFC) | <0.02 % |

7.5% aqueous of sodium carbonate was dosed into this crude sucrose polyester and stirred for 10 minutes at 85°C/200 rpm/1 bar. The batch was then continuously centrifuged at 9600 rpm/85°C and then continuously spray-dried under 20 mbar at 85°C. This batch was further refined by the following procedure. 2 grams of water per kg of spray-dried sucrose polyester product were added and stirred intensively for 10 minutes at 85°C under atmospheric pressure. 5 grams of adsorbent (Trisyl having water content not less than 30% and preferably 60-70% ex Davison Chemical Division of W.R. Grace & Co[R]) per kg of this mixture were dosed to this mixture and agitated intensively for 30 minutes under atmospheric pressure. This mixture was then dried under 100 mbar at 85°C.

After drying, 10 grams of acidic carbon (CA1 ex Norit[R]) per kg of Trisyl treated sucrose polyester were dosed and stirred for 30 minutes at 85°C/1 bar. This mixture was filtered over a sieve filter. Another 10 grams of acidic carbon (CA1 ex Norit[R]) per kg of CA1 treated sucrose polyester were added and stirred for 30 minutes at 85°C under atmospheric pressure. This mixture was then filtered over a sieve filter.

This batch of sucrose polyester was then distilled continuously in a falling film evaporator at 240°C under 4 mbar followed by continuous deodorization in a packed column at 235°C/5 mbar.

This batch of deodorized sucrose polyester was dissolved in hexane and was percolated over a bed of silica at 20°C and after percolation hexane was evaporated fully to recover purified sucrose polyester.

| Level of IMW Components | SFC % |
|---|---|
| After silica column percolation and hexane evaporation | <0.02% |

EXAMPLE 9

A batch of crude sucrose fatty ester polyester synthesized and having the composition according to Example 4 was partially refined according to the process of Example 4, with the exception that after acid treatment and centrifugation, the mixture was washed with 10% water for 10 minutes and centrifuged again for 15 minutes at 3500 rpm at 80°C in order to separate the water phase.

| After pretreatment | <0.02% | <0.02% |
|---|---|---|

This partially refined sucrose polyester was further refined by two different procedures.

Procedure 1 : Dry bleach

15 grams of bleaching earth (Filtrol ex Harshaw/Filtrol, Engelhard[R]) per kg of partially refined sucrose polyester were dosed and stirred for 30 minutes at 1790 rpm/90°C/1 bar and 30 minutes under 50 mbar. The mixture was then filtered over a 2 $\mu$m filter.

| After Filtrol treatment/filtration | 0.3% | 0.06% |
|---|---|---|
| After deodorisation | 0.6% | 0.10% |

Procedure 2 : Dry bleach

Same procedure as Procedure 1, except for dosing 70 grams of bleaching earth (Filtrol)

| After Filtrol treatment/filtration | 0.3% | 0.06% |
|---|---|---|
| After deodorisation | 0.5% | 0.10% |

## Claims

1. A process for refining crude sucrose fatty acid polyester reaction product, comprising the step of subjecting the reaction product to a bleaching treatment, characterized in that it comprises the step of providing a refined polyester material wherein the level of intermediate molecular weight components is less than 0.2 % calculated by weight of said polyester, said intermediate molecular weight being defined as between 0.4 and 0.65 times the molecular weight of the fully esterified sucrose fatty acid polyester.

2. The process of claim wherein the level of intermediate molecular weight components is less than 0.1 % by weight.

3. The process of claim 1 or 2, wherein said bleaching treatment comprises the use of at least one of the following bleaching agents:
   (a) mild acid activated bleaching earth, used in the substantial absence of water;
   (b) thermally activated bleaching earth, used in the substantial absence of water;
   (c) non-activated silica material;
   (d) acid activated carbon, used in the substantial absence of water.

4. The process of claim 3, wherein the bleaching agent (a), (b) or (c) has a pH, as a 10% aqueous slurry, of at least about 3.75, preferably at least 4.0.

5. The process of claim 4, comprising the use of a non-activated silica material.

6. The process of any of claims 1-5, further comprising one or more distilling steps.

7. The process of claim 6, comprising two or more bleaching treatments, at least one of which is carried out after said distilling steps.

8. The process of any of claims 1-7, wherein a refined polyester material is provided having a p-anisidine value of 5 or lower.

9. The process of any on claims 1-8, wherein 0.5 to 3 % of bleaching agent is used by weight of the crude reaction product.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A,D | EP-A-0 434 119 (UNILEVER NV) <br> * examples * <br> --- | 1,3 | C07H13/06 |
| A,D | EP-A-0 435 364 (UNILEVER NV) <br> * example 2 * <br> --- | 1,3 | |
| A | EP-A-0 424 066 (THE PROCTER & GAMBLE COMPANY) <br> * examples * <br> --- | 1,3 | |
| A | EP-A-0 459 172 (DAI-ICHI KOGYO SEIYAKU) <br> * example 1 * <br> * claim 1 * <br> ----- | 1,3 | |

|  |  |
|---|---|
|  | TECHNICAL FIELDS SEARCHED (Int. Cl.5) |
|  | C07H |

The present search report has been drawn up for all claims

| Place of search | Date of completion of the search | Examiner |
|---|---|---|
| THE HAGUE | 13 AUGUST 1993 | SIEMENS T. |